# EUROPEAN PATENT SPECIFICATION

(11) **EP 1 274 407 B1**
(45) Date of publication and mention of the grant of the patent: **08.03.2006**
(21) Application number: 00927425.9
(22) Date of filing: 17.04.2000
(51) Int. Cl.: A61K 31/00, A61K 31/4439, A61P 11/00, A61P 11/06, A61P 17/00, A61P 17/06, A61P 17/08, A61P 17/10, A61P 17/16, A61P 19/02, A61P 19/08, A61P 1/00, A61P 9/10, A61P 13/12, A61P 31/18, A61P 37/06, A61P 37/08, A61P 35/00, A61P 35/02, A61P 3/10, A61P 21/04

(54) **USES OF PPAR-GAMMA AGONISTS IN NEUTROPHIL-INDUCED DISEASES**
VERWENDUNGEN VON PPAR-GAMMA-AGONISTEN IN NEUTROPHIL-VERURSACHTEN ERKRANKUNGEN
UTILISATION D'AGONISTES PPAR-GAMMA DANS LE TRAITEMENT DE MALADIES INDUITES PAR LES NEUTROPHILES.

(30) Priority: 15.04.1999 GB 9908647
(43) Date of publication of application: 15.01.2003
(60) Divisional application: 05076787.0
(73) Proprietor: SMITHKLINE BEECHAM PLC, Brentford, Middlesex TW8 9GS (GB)
(72) Inventor: MACPHEE, Colin Houston, SmithKline Beecham Pharm., Harlow, Essex CM19 5AW (GB)
(74) Representative: Rutter, Keith
(86) International application number: PCT/GB2000/001499
(87) International publication number: WO 2000/062766

(56) References cited:
- WO-A-97/46238
- WO-A-98/25598
- WO-A-98/29113
- US-A- 6 028 088
- INOUE ET AL.: "Troglitazone has a scavenging effect on reactive oxygen species" BIOCHEM. BIOPHYS. RES. COMMUN., vol. 235, no. 1, 1997, pages 113-116, XP001002106
- CHANG T H ET AL: "Induction of differentiation by ligands of PPAR-gamma in non-small cell lung cancer." PROC.AM.ASSOC.CANCER RES. (40, 90 MEET., 184, MARCH 1999) ISSN: 0197-016X, XP001004806 Nat.Cancer-Inst.Rockville
- MOMOI A ET AL: "Thiazolidinedione inhibits production of RANTES in a cytokine-treated human lung epithelial cell line." FEBS LETT. (452, NO. 3, 301-04, 1999) 4 FIG. 28 REF. CODEN: FEBLAL ISSN: 0014-5793, XP001004587 Univ.Kagawa
- YAMASHITA, MASAMICHI (1) ET AL: "Inhibition by troglitazone of antigen-induced leukotriene production by RBL-2H3 cells." JAPANESE JOURNAL OF PHARMACOLOGY, (1999) VOL. 79, NO. SUPPL. 1, PP. 57P. MEETING INFO.: 72ND ANNUAL MEETING OF THE JAPANESE PHARMACOLOGICAL SOCIET SAPPORO, JAPAN MARCH 22-25, 1999 JAPANESE PHARMACOLOGICAL SOCIETY., XP001004803
- DATABASE WPI Week 199831 Derwent Publications Ltd., London, GB; AN 1998-207140 XP002170697 & JP 10 139665 A (SANKYO COMPANY LIMITED), 26 May 1998 (1998-05-26)
- GREENE ET AL.: "Isolation of the peroxisome proliferator activated receptor gamma cDNA: Expression in hematopoietic cells and chromosomal mapping" GENE EXPRESSION, vol. 4, no. 4-5, 1995, pages 281-299, XP000576446
- FUJIMURA ET AL.: "Effects of troglitazone on the growth and differentiation of hematopoietic cell lines" INT. J. ONCOL., vol. 13, no. 6, 1998, pages 1263-1267, XP001004724
- LAMBE, KEVIN G. ET AL: "A human peroxisome-proliferator-activated receptor-gamma is activated by inducers of adipogenesis, including thiazolidinedione drugs." EUROPEAN JOURNAL OF BIOCHEMISTRY, (1996) VOL. 239, NO. 1, PP. 1-7., XP002062380
- MARX ET AL.: "PPAR-gamma in human vascular smooth muscle cells: inhibition of matrix metalloprotease expression, activity and cell migration" CIRCULATION, vol. 98, no. 17 suppl., 27 October 1998 (1998-10-27), pages i382-i383, XP001006186
- ASOU ET AL.: "Growth inhibition of myeloid leukemia cells by troglitazone, a ligand for peroxisome proliferator activated receptor gamma (PPAR-gamma) and retinoids" BLOOD, vol. 90, no. 10 suppl. 1, pt 2, 1997, page 284b XP002062384

## Description

This invention relates to a medicament for the treatment of a disease or condition (associated with increased numbers of neutrophils and/or over-activation of neutrophils, which is chromic obstructive pulmonary disease (COPD).

European Patent Application, Publication Number 0,306,228 relates to certain thiazolidinedione derivatives disclosed as having antihyperglycaemic and hypolipidaemic activity. One particular thiazolidinedione disclosed in EP 0306228 is 5-[4-[2-(N-methyl-N-(2-pyridyl)amino)ethoxy]benzyl]thiazolidine-1,4-dione (hereinafter Compound (I)'). WO94/05659 discloses certain salts of Compound (I) including the maleate salt at example 1 thereof.

Compound (I) is an example of a class of anti-hyperglycaemic agents known as "glitazones" or "thiazolidinedione".

European Patent Applications, Publication Numbers: 0008203, 0139421, 0032128, 0428312, 0489663, 0155845, 0257781, 0208420, 0177353, 0319189, 0332331, 0332332, 0528734, 0508740; International Patent Application, Publication Numbers 92/18501, 93/02079, 93/22445 and United States Patent Numbers 5104888 and 5478852, also disclose certain thiazolidinediones.

It is known that the y-isoform of peroxisome proliferator-activated receptor (herein after PPARγ) is member of a nuclear receptor superfamily that includes receptors for the steroid, thyroid and retinoid hormones (Evans, Science 240, 889-895, (1988)). It is also known from Chawla *et al* that PPARγ is expressed early during the differentiation of adipocytes (Endocrinology 135,798-800, 1994). It is known from J. Biol. Chem., 270,12963-12966 that thiazolidinediones such as Compound (I) are PPARγ agonists.

We have now demonstrated that PPARγ expression is significantly up-regulated in activated neutrophils. Thus, PPARγ agonists are expected to inhibit activated neutrophil function and therefor to have potential use in diseases and conditions where suppression of neutrophil activity or numbers would be of benefit. Such disorders include those listed herein including: gout, arthritis, asthma, chronic obstructive pulmonary disease, irritable bowel syndrome, psoriasis and acne.

Also neutrophils are a major cell type infiltrating the skin dermis and epidermis layer following moderate to server UV sun exposure. These cells have the capability to cause tissue damage and in certain severe circumstances delayed healing of the skin. Thus PPARγ agonists are considered to be useful in preventing and/or treating damage to the skin dermis and/or epidermis layers caused by excess ultra violet radiation or sun-light exposure. They are also considered to aid healing of the skin following such damage.

Accordingly, the invention provides a medicament for the treatment of a disease or condition associated with increased numbers of neutrophils and/or neutrophil over-activation in a mammal such as a human, which medicament comprises an effective, non-toxic and pharmaceutically acceptable amount of a PPARγ agonist, such as Compound (I), to be administered to a mammal in need thereof.

The PPARγ agonist is Compound (I) or a pharmaceutically acceptable derivative thereof.

It will be understood that the PPARγ agonist Compound (I), is to be administered in a pharmaceutically acceptable form including pharmaceutically acceptable derivatives.

Suitable pharmaceutically derivatives include pharmaceutically acceptable salts, esters and solvates, as appropriate to the relevant PPARγ agonist.

Suitable pharmaceutically acceptable salted and or solvated forms of Compound (I) include those described in EP 0306228 and WO94/05659. A preferred pharmaceutically acceptable salt of Compound (I) is a maleate. A preferred pharmaceutically acceptable solvate of Compound (I)is a hydrate.

Other pharmaceutically acceptable derivatives are those disclosed in standard reference texts such as the British and US Pharmacopoeias, Remington's Pharmaceutical Sciences (Mack Publishing Co.) and Martindale The Extra Pharmacopoeia (London, The Pharmaceutical Press) or the above mentioned publications.

The PPARγ agonists may be prepared using known methods, for example those disclosed in the above mentioned publications.

Compound (I) and pharmaceutically acceptable forms thereof may be prepared using known methods, for example those disclosed in EP 0306228 and WO94/05659 publications.

Compound (I) may exist in one of several tautomeric forms, all of which are encompassed by the term Compound (I) as individual tautomeric forms or as mixtures thereof. Compound (I) contains a chiral carbon atom, and hence can exist in up to two stereoisomeric forms, the term Compound (I) encompasses all of these isomeric forms whether as individual isomers or as mixtures of isomers, including racemates.

As used herein the term 'pharmaceutically acceptable' embraces both human and veterinary use: for example the term 'pharmaceutically acceptable' embraces a veterinarily acceptable compound.

For the avoidance of doubt, when reference is made herein to scalar amounts, including mg amounts, of Compound (I) in a pharmaceutically acceptable form, the scalar amount referred to is made in respect of Compound (I) *per se:* For example 2 mg of Compound (I) in the form of the maleate salt is that amount of maleate salt which contains 2 mg of Compound (I).

Neutrophil activation may be demonstrated using conventional methodology such as that disclosed in Current Protocols in Immunology, Vol I, Suppl 1, Unit 6.12.3., the disclosure of which is incorporated herein by reference.

Neutrophils may be isolated from human blood as described in Current Protocols in Immunology Vol I, Suppl 1 Unit 7.23.1, the disclosure of which is incorporated herein by reference.

The medicament of the invention contains the active medicaments in pharmaceutical composition form.

Accordingly, in one aspect the invention provides a pharmaceutical composition for use in the treatment of COPD which is a disease or condition associated with increased numbers of neutrophils and/or neutrophil over-activation in a mammal, such as a human, which composition comprises the PPARγ agonist Compound (I), and a pharmaceutically acceptable carrier therefor.

The compositions may be adapted for any suitable mode of administration, for example oral administration, parenteral administration, sublingual or transdermal administration.

The compositions may be in the form of tablets, capsules, powders, granules, lozenges, suppositories, reconstitutable powders, or liquid preparations, such as oral or sterile parenteral solutions or suspensions.

In order to obtain consistency of administration it is preferred that a composition of the invention is in the form of a unit dose.

Unit dose presentation forms for oral administration may be tablets and capsules and may contain conventional excipients such as binding agents, for example syrup, acacia, gelatin, sorbitol, tragacanth, or polyvinylpyrrolidone; fillers, for example lactose, sugar, maize-starch, calcium phosphate, sorbitol or glycine; tabletting lubricants, for example magnesium stearate; disintegrants, for example starch, polyvinylpyrrolidone, sodium starch glycollate or microcrystalline cellulose; or pharmaceutically acceptable wetting agents such as sodium lauryl sulphate.

The compositions are preferably in a unit dosage form in an amount appropriate for the relevant daily dosage based upon such factors as the particular compound chosen and the nature and severity of the disease or condition.

Suitable dosages including unit dosages of Compound (I), include the known dosages and unit doses for these compounds as described or referred to in reference texts such as the British and US Pharmacopoeias, Remington's Pharmaceutical Sciences (Mack Publishing Co.), Martindale The Extra Pharmacopoeia (London, The Pharmaceutical Press) or the above mentioned publications.

The medicament of the invention the PPARγ agonist may be administered in accordance with know regimens described or referred to in reference texts such as the those referred to above or in the above mentioned publications.

In the treatments the medicaments may be administered from 1 to 6 times a day, but most preferably 1 or 2 times per day.

The solid oral compositions may be prepared by conventional methods of blending, filling or tabletting. Repeated blending operations may be used to distribute the active agent throughout those compositions employing large quantities of fillers. Such operations are of course conventional in the art. The tablets may be coated according to methods well known in normal pharmaceutical practice, in particular with an enteric coating.

Oral liquid preparations may be in the form of, for example, emulsions, syrups, or elixirs, or may be presented as a dry product for reconstitution with water or other suitable vehicle before use. Such liquid preparations may contain conventional additives such as suspending agents, for example sorbitol, syrup, methyl cellulose, gelatin, hydroxyethylcellulose, carboxymethylcellulose, aluminium stearate gel, hydrogenated edible fats; emulsifying agents, for example lecithin, sorbitan monooleate, or acacia; non-aqueous vehicles (which may include edible oils), for example almond oil, fractionated coconut oil, oily esters such as esters of glycerine, propylene glycol, or ethyl alcohol; preservatives, for example methyl or propyl p-hydroxybenzoate or sorbic acid; and if desired conventional flavouring or colouring agents.

For parenteral administration, fluid unit dosage forms are prepared utilizing the compound and a sterile vehicle, and, depending on the concentration used, can be either suspended or dissolved in the vehicle. In preparing solutions the compound can be dissolved in water for injection and filter sterilized before filling into a suitable vial or ampoule and sealing. Advantageously, adjuvants such as a local anaesthetic, a preservative and buffering agents can be dissolved in the vehicle. To enhance the stability, the composition can be frozen after filling into the vial and the water removed under vacuum. Parenteral suspensions are prepared in substantially the same manner, except that the Compound (I)s suspended in the vehicle instead of being dissolved, and sterilization cannot be accomplished by filtration. The compound can be sterilized by exposure to ethylene oxide before suspending in the sterile vehicle. Advantageously, a surfactant or wetting agent is included in the composition to facilitate uniform distribution of the compound.

Compositions may contain from 0.1% to 99% by weight, preferably from 10-60% by weight, of the active material, depending upon the method of administration.

As indicated the PPARγ agonist can also be administered in a pharmaceutically acceptable form suitable for topical application.

Compositions may, if desired, be in the form of a pack accompanied by written or printed instructions for use.

The compositions are formulated according to conventional methods, such as those disclosed in standard reference texts, for example the British and US Pharmacopoeias, Remington's Pharmaceutical Sciences (Mack Publishing Co.) and Martindale The Extra Pharmacopoeia (London The Pharmaceutical Press) (for example see the 31st Edition page 341 and pages cited therein) and Harry's Cosmeticology (Leonard Hill Books) or as disclosed in the above-mentioned publications.

No adverse toxicological effects have been established for the compositions or methods of the invention in the above mentioned dosage ranges.

The following example illustrates the invention but does not limit it in any way.

### DEMONSTRATION OF EFFECT

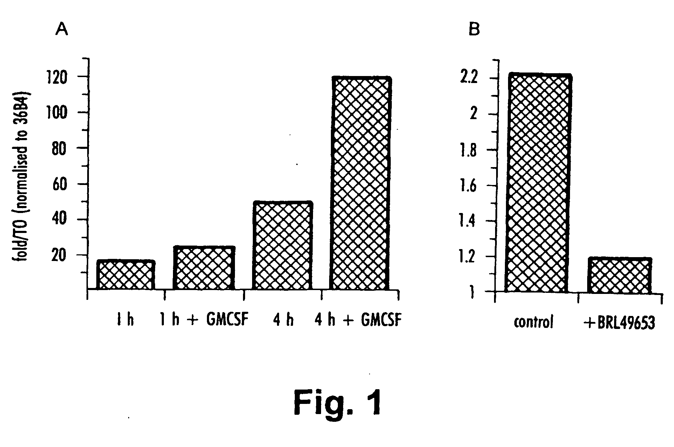

The results shown in Figure 1 demonstrate:
(Figure 1A): That PPARgamma mRNA is rapidly and significantly increased during (data taken at 1 and 4 hours) human neutrophil attachment to culture dishes and is further enhanced by treatment with GM-CSF, (Figure lA).
(Figure 1B): That the increase in Gelatinase B mRNA expression observed 4 hours after neutrophil attachment was reduced by treatment with 1 µM of Compound (I). Time zero (TO) refers to expression in naïve human neutrophils prior to attachment.

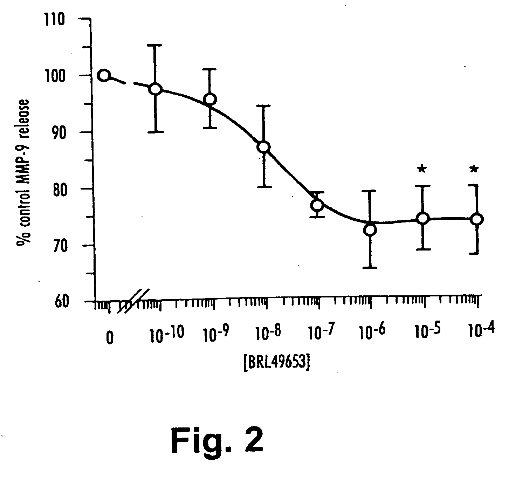

Figure 2: Inhibition of MMP-9 release by Compound (I) Neutrophils were isolated by countercurrent centrifugal elutriation to a purity of at least 98 % and were maintained in RPMI 1640 plus 1 % BSA. Cells were cultured in 48 well plates and attached to the wells within 1 hour of plating. Compound (I) (referred to as "BRL49653" in Figure 2) was included from the beginning of the experiment. After 4 hours the media was removed and a human MMP-9 ELISA assay (Amersham) was performed. Data (from 4 different individuals) were not suitable for pooling directly, so for the purposes of the figure they were normalised to percentage of no compound control. Asterisks denote p < 0.05 using a paired Student's t-test performed on the raw data prior to normalisation. IC50 for the inhibition by Compound (I) of MMP-9 release was 10.6 nM (calculated by fitting a 4-parameter logistic curve using Statistica).

## Claims

1. The use of 5-[4-[2-(N-methyl-N-(2-pyridyl)amino)ethoxy]benzyl]thiazolidine-2,4-dione ("Compound (I)") or a pharmaceutically acceptable derivative thereof for manufacture of a medicament for the treatment of chronic obstructive pulmonary disease (COPD).

## Revendications

1. Utilisation de 5-[4-[2-(N-méthyl-N-(2-pyridyl)-amino)éthoxy]benzyl]thiazolidine-2,4-dione ("Composé (I)") ou d'un de ses dérivés pharmaceutiquement acceptables pour la production d'un médicament destiné au traitement de la maladie pulmonaire obstructive chronique (COPD).

## Patentansprüche

1. Verwendung von 5-[4-[2-(N-Methyl-N-(2-pyridyl)amino)ethoxy]benzyl]thiazolidin-2,4-dion ("Verbindung (I)") oder einem pharmazeutisch verträglichen Derivat davon zur Herstellung eines Medikaments zur Behandlung von chronisch obstruktiver Lungenerkrankung (COPD).
